# EUROPEAN PATENT APPLICATION

(11) **EP 3 928 691 A1**
(43) Date of publication of application: **29.12.2021**
(21) Application number: 20182661.7
(22) Date of filing: 26.06.2020
(51) Int. Cl.: A61B 5/042, A61B 5/053, A61B 5/06, A61B 5/00, G06K 9/62

(54) **IMAGING A HOLLOW ORGAN**

(71) Applicant: Koninklijke Philips N.V., 5656 AG Eindhoven (NL)
(72) Inventor: SCHWARTZ, Yitzhack, 5656 AE Eindhoven (NL); IBRAGIMOV, Zalman, 5656 AE Eindhoven (NL); BEN DAVID, Yehonatan, 5656 AE Eindhoven (NL); DICHTERMAN, Eli, 5656 AE Eindhoven (NL)
(74) Representative: Philips Intellectual Property & Standards

(57) **Abstract**

The present invention relates to imaging a hollow organ. In order to provide an improved and facilitated imaging of a hollow organ of interest, a device (10) for providing three-dimensional data of a hollow organ is provided that comprises a measurement input (12), a data processor (14) and an output interface (16). The measurement input is configured to receive a plurality of local electric field measurements (18) of at least one electrode on a catheter inserted in a lumen of a hollow organ of interest. The measurement input is also configured to receive geometrical data (20) representative of the location of the at least one electrode inside the lumen during the measurements. The data processor is configured to receive pre-set electric field characteristics (22) associated with predetermined anatomical landmarks of the hollow organ expectable in the lumen in dependency of a type of the hollow organ. The data processor is also configured to compare at least one of the plurality of local electric field measurements with the pre-set electric field characteristics to determine matching electric field measurements. The data processor is further configured to allocate local electric field measurements to matching electric field characteristics based on the geometrical data to identify anatomical landmarks of the hollow organ by identifying those local field measurements in the plurality of measurements that correspond to landmarks of the hollow organ. The data processor is still further configured to generate a three-dimensional image data cloud (24) by transforming the allocated electric field measurements into portions of the three-dimensional image data cloud based on the identified anatomical landmarks. The output interface is configured to provide the three-dimensional image data cloud.

## Description

### FIELD OF THE INVENTION

The present invention relates to a device for providing three-dimensional data of a hollow organ, to a system for measuring a hollow organ and to a method for providing three-dimensional data of a hollow organ.

### BACKGROUND OF THE INVENTION

For examination and interventional purposes, visualizations are provided of an inner side enclosing a lumen of a hollow organ. For example, heart chambers may be visualized as projections to provide a better understanding of their physiological and geometrical structure. As an example, pre-operative CT-data is provided to generate a 3D model of the heart. During an intervention, live images can be combined with an adaption of the 3D model to identify anatomical structures. Another example is a flat projection of the 3D image data and the manual identification of landmarks. However, it has been shown that this is cumbersome for the user due to preparation effort. Also, the presentation is not provided in real-time.

### SUMMARY OF THE INVENTION

There may thus be a need to provide an improved and facilitated imaging of a hollow organ of interest.

The object of the present invention is solved by the subject-matter of the independent claims; further embodiments are incorporated in the dependent claims. It should be noted that the following described aspects of the invention apply also for the device for providing three-dimensional data of a hollow organ, for the system for measuring a hollow organ and for the method for providing three-dimensional data of a hollow organ.

According to the present invention, a device for providing three-dimensional data of a hollow organ is provided. The device comprises a measurement input, a data processor and an output interface. The measurement input is configured to receive a plurality of local electric field measurements of at least one electrode on a catheter inserted in a lumen of a hollow organ of interest. The measurement input is also configured to receive geometrical data representative of the location of the at least one electrode inside the lumen during the measurements. The data processor is configured to receive pre-set electric field characteristics associated with predetermined anatomical landmarks of the hollow organ expectable in the lumen in dependency of a type of the hollow organ. The data processor is also configured to compare at least one of the plurality of local electric field with the pre-set electric field characteristics to determine matching electric field measurements. The data processor is further configured to allocate local electric field measurements to matching electric field characteristics based on the geometrical data to identify anatomical landmarks of the hollow organ by identifying those local field measurements in the plurality of measurements that correspond to landmarks of the hollow organ. The data processor is still further configured to generate a three-dimensional image data cloud by transforming the allocated electric field measurements into portions of the three-dimensional image data cloud based on the identified anatomical landmarks. The output interface is configured to provide the three-dimensional image data cloud.

This provides the advantage that the inner side enclosing the hollow organ is determined with less effort. A live presentation is also enabled.

The "device for providing three-dimensional data of a hollow organ" can also be referred to as "device for generating a three-dimensional data cloud of a hollow organ" or as "device for mapping a hollow organ" or as "device for imaging a hollow organ".

In an example, the portions of the three-dimensional image data cloud comprise surface portions as they relate to portions of the inner surface of the hollow organ.

The portions of the three-dimensional image are thus based on the idea of generating image data comprising a three-dimensional representation of at least part of the hollow organ where the three-dimensional representation comprises an indication of the identified anatomical landmark. The portions, or surface portions would then be the indication of those findings.

According to an example, the data processor is configured to project a representation of an inner surface enclosing the lumen as a 3D view based on the three-dimensional image data cloud.

According to an example, the data processor is configured to project a flattened 3D panoramic view of the inner surface enclosing the lumen based on the three-dimensional image data cloud. The flattened 3D panoramic view shows at least some surface parts of the inner surface. The data processor is also configured to provide a preset feature of interest in a center region of the flattened 3D panoramic view.

According to an example, the data processor is configured to generate the flattened 3D panoramic view based on user selected criteria. The selected criteria comprise a type of operation or treatment of the hollow organ.

In an option, for finding a projection according to the user selected criteria, the data processor is configured to arrange a plurality of different landmark related criteria in different weighting orders depending on the user selected criteria and to check if these criteria are fulfilled according to the respective weighting order for variations of projections.

According to an example, a display is provided configured to display the 3D view and/or the flattened 3D panoramic view.

According to an example, the pre-set electric field characteristics comprise at least one of the group of i) predefined gradients describing changes in the electric field, and ii) predefined patterns describing attributes of the electric field. For the comparison, the data processor is configured to match the measured electric field values with the pre-set electric field characteristics to identify measurements relating to a location of a landmark.

According to the present invention, also a system for measuring a hollow organ is provided. The system comprises a catheter with at least one electrode. The system also comprises an electric field generator and a device for providing three-dimensional data of a hollow organ according to one of the preceding examples. The catheter is configured to be partly inserted into the lumen and moved inside the lumen. The catheter is also configured to conduct a plurality of local electric field measurements inside the lumen with the at least one electrode. The catheter is further configured to provide the local electric field measurements data to the measurement input of the device for providing three-dimensional data of a hollow organ. The electric field generator is configured to generate at least one electric field inside a lumen of a hollow organ. The electric field generator is configured to provide the geometrical data representative of the location of the at least one electrode inside the lumen during the measurements to the measurement input of the device for providing three-dimensional data of a hollow organ.

The "system for measuring a hollow organ" can also be referred to as "system for determining a hollow organ" or as "system for imaging a hollow organ".

According to an example, the catheter is configured to be inserted into a heart comprising several chambers as the hollow organ, from which heart at least one chamber is to be imaged. In an alternative or additional example, the catheter is configured to be inserted into at least one of the group of a bladder, a uterus, a stomach and a colon.

According to the present invention, also a method for providing three-dimensional data of a hollow organ is provided. The method comprises the following steps:
- receiving a plurality of local electric field measurements of at least one electrode on a catheter inserted in a lumen of a hollow organ of interest;
- providing geometrical data representative of the location of the at least one electrode inside the lumen during the measurements;
- providing pre-set electric field characteristics associated with predetermined anatomical landmarks of the hollow organ expectable in the lumen in dependency of a type of the hollow organ;
- comparing at least one of the plurality of local electric field measurements with the pre-set electric field characteristics to determine matching electric field measurements;
- allocating local electric field measurements to matching electric field characteristics based on the geometrical data to identify anatomical landmarks of the hollow organ by identifying those local field measurements in the plurality of measurements that correspond to landmarks of the hollow organ;
- generating a three-dimensional image data cloud by transforming the allocated electric field measurements into portions of the three-dimensional image data cloud based on the identified anatomical landmarks; and
- outputting the three-dimensional image data cloud.

The "method for providing three-dimensional data of a hollow organ" can also be referred to as "method for generating a three-dimensional data cloud of a hollow organ" or as "method for mapping a hollow organ" or as "method for imaging a hollow organ".

According to the present invention, also a computer program enabling a processor to carry out the method of one of the examples above is provided.

In an example, a computer program or program element for controlling an apparatus according to one of the examples above is provided, which program or program element, when being executed by a processing unit, is adapted to perform the method steps of one of the method example above.

According to the present invention, also a computer readable medium having stored the program element of the examples of the computer program is provided.

According to an aspect, the landmarks are determined based on expected phenomena and a panoramic view is generated on the fly. The panoramic view is created in front of the user's eye. To base the landmark recognition on measurement fragments and the continuous growing of the three-dimensional image data cloud, also allows to apply landmark detection in case of abnormalities. Contrary to a model-based approach, the output image is based on measurements and does not even out abnormalities or even deviations. Only what is measured is taken into account. Other parts remain undetected until they are also measured. This enables an on-the-fly generating of the projection in real-time, or at least in near-real-time with only a small timely displacement, like less than two seconds.

In an example, the three-dimensional image data cloud is continuously growing. For example, the cloud is updated every two seconds or less and an image is provided in a stepped manner. The catheter is used in real-time.

In an example, the data cloud is an incomplete data cloud. For example, the cloud contains, i.e. is built up of the landmarks as surface portions. Other parts may still remain non-defined.

As an option, the data cloud is built up on the fly and doesn't need to be complete. For example, depending on the movement of the electrodes for measuring inside the hollow organ, the data cloud is provided respectively in parts only. The more measures and movement of the electrodes are provided, the more data is generated and the cloud is gradually generated.

In an example, for providing an optimum view, a segmentation of the determined image parts is provided and updated accordingly. Thus, a partial understanding is provided that continuously grows to a complete understanding.

According to a further aspect, forward oriented measurements are provided. Based on the detected measurements, and based on the forward directed electric field measurements, it is achieved to identify landmarks like the connected veins in a heart chamber even before they are reached.

According to an aspect, the panoramic view is provided during the measurements. For example, the panoramic view is provided right from the start. It may be less detailed when started, but is continuously growing. In an example, the panoramic view is generated even if this is shown only partially. Thus, an interim outcome of an imaging process is provided.

In example, a point is tagged in 3D space, such as dimples in an atrial view when continuously forming the anatomy.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a device for providing three-dimensional data of a hollow organ.
Fig. 2 schematically shows an example of a system for measuring a hollow organ.
Fig. 3 shows basic steps of an example of a method for providing three-dimensional data of a hollow organ.
Fig. 4 shows another example of a method for providing three-dimensional data of a hollow organ.
Fig. 5 shows an example of an interim state of a three-dimensional image data cloud with measurements used for identifying anatomical landmarks.
Fig. 6 shows an example of a setup for identifying a preferred viewing angle for projecting a flattened 3D view.
Fig. 7 shows an example of a flattened panoramic view.
Fig. 8 shows another example of a flattened panoramic view.
Fig. 9 shows a further example of a flattened panoramic view.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

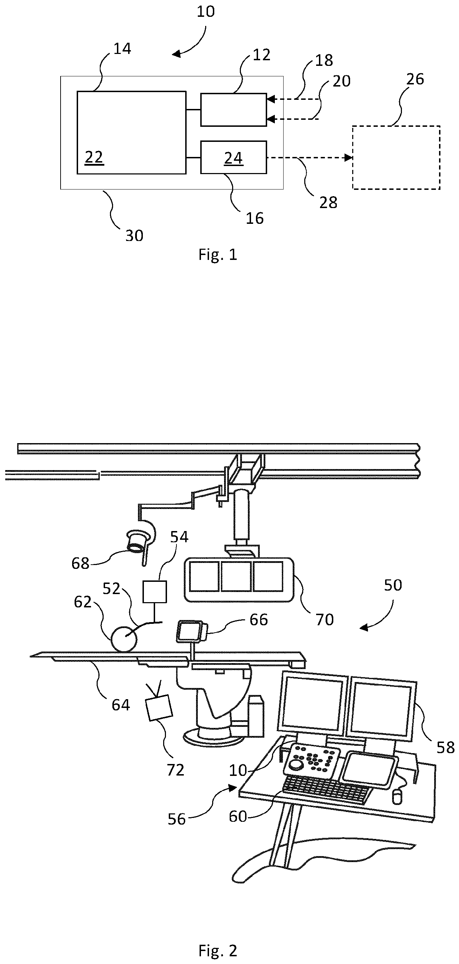

Fig. 1 schematically shows an example of a device 10 for providing three-dimensional data of a hollow organ. The device comprises a measurement input 12, a data processor 14 and an output interface 16. The measurement input 12 is configured to receive a plurality of local electric field measurements 18 of at least one electrode on a catheter inserted in a lumen of a hollow organ of interest. The measurement input 12 is configured to receive geometrical data 20 representative of the location of the at least one electrode inside the lumen during the measurements. The data processor 14 is configured to receive pre-set electric field characteristics 22 associated with predetermined anatomical landmarks of the hollow organ expectable in the lumen in dependency of a type of the hollow organ. The data processor 14 is also configured to compare at least one of the plurality of local electric field measurements with the pre-set electric field characteristics to determine matching electric field measurements. The data processor 14 is further configured to allocate local electric field measurements to matching electric field characteristics based on the geometrical data to identify anatomical landmarks of the hollow organ by identifying those local field measurements in the plurality of measurements that correspond to landmarks of the hollow organ. The data processor 14 is still further configured to generate a three-dimensional image data cloud 24 by transforming the allocated electric field measurements into portions of the three-dimensional image data cloud based on the identified anatomical landmarks. The output interface 16 is configured to provide the three-dimensional image data cloud 24.

The output interface 16 outputs the three-dimensional image data cloud 24 for further data processing, for example.

In an example, several electrodes are provided on a catheter, e.g. a catheter tip in a fixed inter-electrode spacing. The spacing together with a predetermined electrical weight length of each electrode are used as an internal ruler for the measurements.

In an example, a set of pre-set electric field characteristics is provided.

The term "anatomical landmark" refers to identifiable features inside the lumen. The hollow organ can be a heart, for example, and the lumen can be a heart chamber. The anatomical landmarks would then be, for example, atrio-ventricular and ventriculo-arterial valves, venous ostia, atrial appendages and ridges, an aortic root, and the like. The anatomical landmarks thus refer to distinguishable portions or parts of the organ that enclose the lumen.

The term "type" of the hollow organ relates to what organ is imaged, e.g. if a heart, bladder, uterine, stomach or colon is imaged.

The hollow organ comprises at least one lumen to be imaged. The lumen is, for example, at least partly spherically shaped.

The electric fields are also referred to as electrical fields.

The local electric field measurements provide at least a voltage measurement. In an example, the local electric field measurements comprise only voltage measurements, such as measuring the change of voltage or delta in the voltage between two locations of the electrodes when moving inside the lumen. In another example, the local electric field measurements comprise impedance measurements.

The voltage measurements are then transferred into a distance value. The distance values are then used for generating three-dimensional data.

The term "geometrical data" relates to, for example, spatial information regarding the location of the at least one electrode on the catheter when performing the electric field measurements. This can be provided in many ways. The geometrical data can also be referred to as spatial information. The geometrical data can also be referred to as geometrical considerations. The term "geometrical data" can also be referred to as spatial information.

The geometrical data can be provided in a direct or indirect manner. The data can thus be provided, i.e. taken, directly or indirectly. For example, the geometrical data can be provided directly as geometric detection or measurements, e.g. by a tracking system. For example, the geometrical data can also be provided indirectly by taking other sources of information and to compute the spatial data based on other kinds of information, such as based on image data.

In an example, the geometrical data are measured data. In other options, provided in addition or alternatively, the geometrical data are provided as predetermined data when the spatial relations are predefinable, for example. In another example, the geometrical data are provided as predefined data.

In an example, the geometrical data relates to the location of the electrode. For example, the geometrical data comprises the actual location data of the electrode when performing the electrical field measurements. As an example, the position of the electrode/s is/are tracked. As another example, the movement of the electrode/s is/are tracked.

For example, the geometrical data are provided as a starting point, i.e. as reference to build up the three-dimensional data cloud.

In an example, by the matching operation, a number of measurements is achieved that have a "counterpart" in the pre-set characteristics. As far as these represent a landmark, the matching measurements can be identified to correspond to the respective landmark of the organ.

In an example, the data processor 14 is configured to project a representation of an inner surface enclosing the lumen as a 3D view based on the three-dimensional image data cloud.

In an example, the three-dimensional data 24 is building up a partial mesh with mesh fragments. The mesh parts are then transferred into a surface.

In an example, the data processor 14 is configured to project a flattened 3D panoramic view of the inner surface enclosing the lumen based on the three-dimensional image data cloud, the flattened 3D panoramic view showing at least some surface parts of the inner surface. The data processor 14 is also configured to provide a preset feature of interest in a center region of the flattened 3D panoramic view.

The feature of interest may be selected as one of the predetermined landmarks.

The feature of interest is defined by a planned operation.

In an example, the system knows a number of views and the user can select one of the views. Upon selection, the system provides the according flattened 3D panoramic view.

The system identifies the pre-set features and sets the volume of interest where maximal accuracy is required in the center of the panoramic image to facilitate single-user operability and execution of a planned therapy. Further geometric restraints can be predefined and stored in the system.

In an example, the data processor 14 is configured to generate the flattened 3D panoramic view based on user selected criteria. The selected criteria comprise a type of operation or treatment of the hollow organ.

In an option, for finding a projection according to the user selected criteria, the data processor 14 is configured to arrange a plurality of different landmark related criteria in different weighting orders depending on the user selected criteria and to check if these criteria are fulfilled according to the respective weighting order for variations of projections.

In an example, for the user selected criteria, a plurality of predetermined intervention types is provided for selection by the user. Different viewing criteria are pre-set for the different intervention types.

In an example, the intervention types relate to certain operations that the user is intending to perform and for which the view is needed.

In an example, the three-dimensional image data cloud is arranged in relation to a virtual viewing direction. The degrees of freedom of the three-dimensional image data cloud are controlled such that predefined criteria are met.

The panoramic view can be generated even if not all landmarks are allocated yet. Thus, a panoramic view can be generated on the go, or on the flight.

It is thus possible to provide an image from the beginning.

For finding a suitable projection according to the user selected criteria, a plurality of different landmark related criteria is arranged in different weighting orders depending on the user selected criteria and it is checked if these criteria are fulfilled according to the respective weighting order for variations of projections. The variation with best match is selected. Variations in the viewing direction and projection angles are also provided and entered into a self-learning process to help the system finding an optimal view in which the different landmark related criteria are fulfilled to the largest extent.

In an example, a cutting plane is arranged within the three-dimensional image data cloud. The so-to-speak open sphere is then projected as an elliptic projection. A point of view is defined to then further manipulate the degrees of freedom of movement. The point of view can then also be re-defined. The changes are immediately transformed in the view that is projected. This provides a single-user operability.

In an option, indicted by hashed lines, a display 26 is provided configured to display the 3D view and/or the flattened 3D panoramic view. The display is data connected to the output interface 16, as indicated with hashed connection line 28. The connection can be provided wireless or wire bound.

A frame 30 indicates an option where the measurement input 12, the data processor 14 and the output interface 16 are arranged in a common housing. In a further example, the data processor 14 and the output interface 16 are arranged separately. In a still further example, the data processor 14 and the output interface 16 are arranged in a housing of a system together with other components.

In an example, the pre-set electric field characteristics comprise predefined gradients describing changes in the electric field.

In an example, provided in addition or alternatively, the pre-set electric field characteristics comprise predefined patterns describing attributes of the electric field.

In a further example, the pre-set electric field characteristics are provided upfront as references. This data is provided to the processor. The processor may also store them or has them stored in a storage partition for use during the measurements.

For the comparison, the data processor 14 is configured to match the measured electric field values with the pre-set electric field characteristics to identify measurements relating to a location of a landmark.

Fig. 2 schematically shows an example of a system 50 for measuring a hollow organ. The system 50 comprises a catheter 52 with at least one electrode, an electric field generator 54 and an example of the device 10 for providing three-dimensional data of a hollow organ according to one of the preceding examples. As an option, the device 10 for providing three-dimensional data of a hollow organ is provided in a control arrangement 56, also comprising monitors 58 and user-interface appliances 60 like keyboard, mouse or graphic tablet.

An object 62 of interest with a lumen is schematically indicated, arranged on a subject support 64. A smaller display 66 is provided, e.g. to provide a user control interface. Also, light devices 68 may be provided, and also larger display units 70

The catheter 52 is configured to be partly inserted into the lumen and moved inside the lumen. The catheter 52 is also configured to conduct a plurality of local electric field measurements inside the lumen with the at least one electrode. The catheter 52 is also configured to provide the local electric field measurements data to the measurement input 12 of the device 10 for providing three-dimensional data of a hollow organ. The electric field generator 54 is configured to generate at least one electric field inside a lumen of the hollow organ, and to provide the geometrical data representative of the location of the at least one electrode inside the lumen during the measurements to the measurement input 12 of the device 10 for providing three-dimensional data of the hollow organ.

In an example, the electric field generator 54 is configured to provide one electric field. In another example, the electric field generator 54 is configured to provide two or three electric fields. In a further example, the electric field generator 54 is configured to provide more than three electric fields.

In an example, the catheter 52 comprises a catheter tip portion (not shown in detail), and the at least one electrode is arranged on the catheter tip portion.

In an example, the electric measurements comprise electric signals from the body surface.

In another example, the electric measurements comprise electric signals from the catheter sensors. Catheters with different electrode arrangements are provided.

A combination of local electric field measurements and global electric field measurements are provided. The measured voltage is transformed into the "reality space" (also referred to as Euclidian R-space).

In an example, at least one electrode is tracked in space.

In an example, it is further provided at least one interventional device for insertion into a hollow organ. The interventional device comprises at least one tool for treatment inside the hollow organ. The interventional device further comprises the catheter having at least one electrode. The catheter is provided for guiding the at least one tool inside the hollow organ. The system for measuring a hollow organ is also referred to as a system for interventional treatment inside a hollow organ.

In an example, the geometrical data comprises spatial tracking of the at least one electrode inside the organ during the measurements by the electric field generator.

As an option, a tracker 72 is provided configured to provide spatial information of a starting point for the moving of the catheter, e.g. the catheter tip inside the hollow organ. The spatial tracking is based on the starting point.

For the spatial tracking, a local electric field is generated and a location of the catheter tip is determined for the local electric field.

The "tracker" can be provided as a further imaging system, like ultrasound, to guide the insertion of the catheter and to detect the entry of the catheter tip into the lumen of the hollow organ.

In another example, the "tracker" is provided in form of pre-operational three-dimensional image data and a registration of the current position of the catheter tip in such three-dimensional image data, for example by electromagnetic tracking of the catheter tip.

In a further example, the "tracker" is provided in form of an electric field (also referred to as global electric field) at least in the region of interest and a registration of the electric field with pre-operational three-dimensional image data. The catheter tip is tracked in the electric field (global electric field).

In a still further example, the tracking is achieved by providing a predefined expected change of electric field measurements of the catheter tip and to compare the measurements of the catheter tip with the expected change while moving the catheter tip along a vessel that leads to the hollow organ. When the expected change appears, the starting point is assumed to be the connecting area of the vessel's lumen and the hollow organ's lumen.

In one example, the starting point is the center of the lumen. In another example, the starting point is one of the enclosing walls. In a further example, the starting point is a vessel connection point to the lumen.

In an example, non-vessel organs are to be imaged. For example, the hollow organ has a lumen that is non-elongate, such as spherical, or spherical-like.

In an example, the catheter is configured to be inserted into a heart comprising several chambers as the hollow organ, from which heart at least one chamber is to be imaged.

In another example, provided in addition or alternatively, the catheter is configured to be inserted into at least one of the group of bladder, uterus, stomach and colon.

In an example, electrodes are provided that are reading voltages at a sampling rate of 100 Hz.

In an example, tissue response is time-tracked and an amplitude is measured in form of the peak-to-peak voltage. If the sensors, i.e. the electrodes are moved inside a vessel, no voltage would be measured thus providing the information to be in a vessel.

In an example, certain gradients are expected for certain times. Based on track movement and time tracking, it is possible to differentiate between a vessel and a heart chamber.

In an example, the system can deal with any imaging style. Different visible outcomes can be provided.

In another example, the panoramic view is provided with double decker portions for regions with not enough details to compensate for these encounters.

Once an optimal view is found, it is possible to store this and to go back to this view later on.

In an example, the three-dimensional image data cloud is provided with different images for sections belonging to segmented and identified parts.

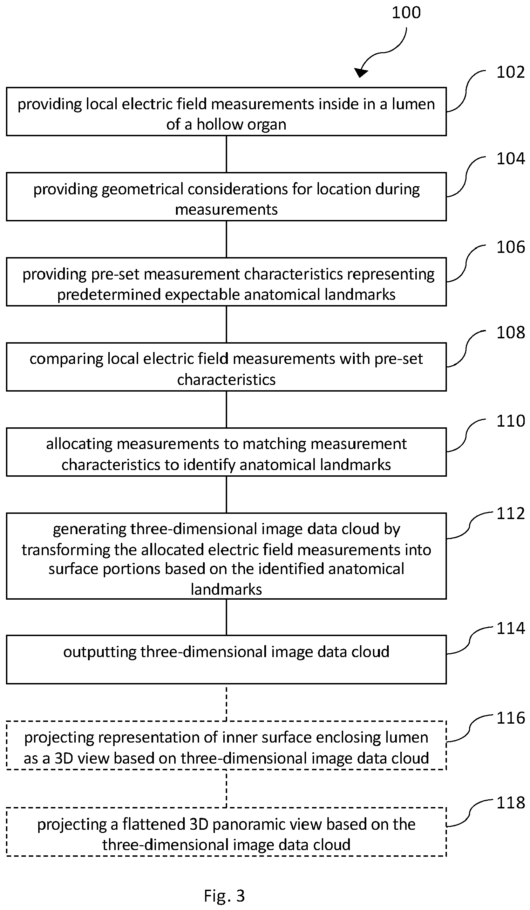

Fig. 3 shows basic steps of an example of a method 100 for providing three-dimensional data of a hollow organ, the method comprising the following steps:
- In a first step 102, also referred to as step a), a plurality of local electric field measurements of at least one electrode on a catheter inserted in a lumen of a hollow organ of interest are received.
- In a second step 104, also referred to as step b), geometrical data representative of the location of the at least one electrode inside the lumen during the measurements are provided.
- In a third step 106, also referred to as step c), pre-set electric field characteristics are provided associated with predetermined anatomical landmarks of the hollow organ expectable in the lumen in dependency of a type of the hollow organ.
- In a fourth step 108, also referred to as step d), at least one of the plurality of local electric field measurements is compared with the pre-set electric field characteristics to determine matching electric field measurements.
- In a fifth step 110, also referred to as step e), local electric field measurements are allocated to matching electric field characteristics based on the geometrical data to identify anatomical landmarks of the hollow organ by identifying those local field measurements in the plurality of measurements that correspond to landmarks of the hollow organ.
- In a sixth step 112, also referred to as step f), a three-dimensional image data cloud is generated by transforming the allocated electric field measurements into portions of the three-dimensional image data cloud based on the identified anatomical landmarks.
- In a seventh step 114, also referred to as step g), the three-dimensional image data cloud is outputted.

The steps of providing the local electric field measurements, and providing the geometrical data, and providing the pre-set electric field characteristics, can also be referred to as receiving the local electric field measurements, and receiving the geometrical data, and receiving the pre-set electric field characteristics.

The first step 102, the second step 104 and the third step 106 can also be provided in a different order or also simultaneously.

The generation of the three-dimensional image data cloud is based on collecting points, i.e. measurements, inside the lumen. This provides a cloud of signals that is transformed into a surface representation of the lumen.

The above brings or transforms voltage measurements into a representation of the reality. The comparing-allocating steps transform micro-voltage to reality, so-to-speak.

In an option, it is further provided a step of inserting a catheter with at least one electrode in a lumen of a hollow organ and moving the at least one electrode inside the lumen. It is also provided a step of conducting a plurality of local electric field measurements inside the lumen with the at least one electrode for providing the local electric field measurements.

The method provides a constant imaging. Since the method does not provide any mapping of the reality to a model, a larger degree of actually providing reality context is achievable.

Moving the catheter forward and taking measurements at the same time means that it is actually looked forward.

The pre-set electric field characteristics are defining structures that are expected for the particular type of organ. In other words, the pre-set electric field characteristics are representing predetermined anatomical landmarks of the hollow organ. The pre-set electric field characteristics are telling the system what can be expected, i.e. what is expected to be encountered by the catheter tip. Allocating the actual measurements to the expected phenomenon allows to gradually build up the three-dimensional image data cloud.

The "pre-set electric field characteristics" can also be referred to as "pre-set associated electric field characteristics". For example, the pre-set electric field characteristics are provided as pre-set electric field measurements. The pre-set electric field characteristics can also be referred to as pre-set electric field measurement characteristics. Simply said, the pre-set electric field characteristics relate to results of measurements that can be expected. In an example, the pre-set electric field characteristics are based on measurements pre-taken and identified as showing identifiable characteristics for certain anatomical features or landmarks.

In an example, the three-dimensional image data cloud is built up during the electric field measurements. The three-dimensional image data cloud is built up as the measurements are made. Based on the characteristics of the fields and the geometrical consideration, the lumen is thus gradually identifiable.

The term "data cloud" refers to points in space for which data is determined, e.g. by comparing at least one of the plurality of local electric field measurements with the pre-set electric field characteristics and allocating of local electric field measurements to matching electric field characteristics based on the geometrical data to identify anatomical landmarks of the hollow organ. The data cloud can also be referred to as spatial data collection or spatial data pile or data formation. Since the data is formed relating to an actual three-dimensional structure, and the data is thus representing such spatial arrangement, the data cloud can also be referred to as spatial data formation. The term "cloud" refers to the continuously forming of a region for which data is provided. Since the identified locations are provided as individual identifications that continuously provides relating data, the forming reminds of clouds building up for example by having a plurality of individual starting points.

As an example, anatomical variations are detected by the measurements and are present in the three-dimensional image data cloud.

It is noted that the three-dimensional image data cloud is relying on a non-model based approach.

The method is based on geometrical data and the measured electric characteristics.

In Fig. 3, hashed lines indicate a further option, according to which it is further provided a step 116 of projecting a representation of an inner surface enclosing the lumen as a 3D view based on the three-dimensional image data cloud.

In Fig. 3, in addition, or alternatively, it is further provided a step 118 of projecting a flattened 3D panoramic view of the inner surface enclosing the lumen based on the three-dimensional image data cloud, the flattened 3D panoramic view showing at least some surface parts of the inner surface. A preset feature of interest is provided in a center region of the flattened 3D panoramic view.

In another example, a method for imaging a hollow organ is provided. The method comprises the steps of: inserting a catheter with at least one electrode in a lumen of a hollow organ and moving the at least one electrode inside the lumen; conducting a plurality of local electric field measurements inside the lumen with the at least one electrode; providing geometrical data representative of the location of the at least one electrode inside the lumen during the measurements; providing a set of pre-set electric field characteristics associated with predetermined anatomical landmarks of the hollow organ expectable in the lumen in dependency of a type of the hollow organ; comparing at least one of the plurality of local electric field measurements with the pre-set electric field characteristics; allocating local electric field measurements to matching electric field characteristics to identify anatomical landmarks of the hollow organ; generating a three-dimensional image data cloud by transforming the electric field measurements into portions based on the identified anatomical landmarks; and providing the three-dimensional image data cloud.

In an example, an electro-anatomical imaging system is provided that utilizes electromagnetic dielectrics for intra-body localization and tracking. The system can operate with electrophysiology (EP), diagnostic or therapeutic catheters as well as pacing leads, guidewires and deflectable sheaths with electrodes.

As an example, the system is configured for cardiac electrophysiologists and structural heart disease specialists who rely on detailed anatomy for diagnosis, preplanning and execution of a large variety of catheter-based therapies. The system can accordingly acquire highly detailed "CT-like" images of all the cardiac chambers with their characteristic anatomical landmarks. However, the imaging is provided without the need for applying X-ray radiation.

The images can be displayed as either a conventional full 3D reconstruction or as novel flattened 3D panoramic view. In an option, the panoramic view can be manually manipulated to match specific objectives per planned procedure. To best function, in an example, it enables self-explanatory spatial orientation, identification of all relevant anatomical landmarks (or anatomical variations) and execution of a planned procedure.

An optimal panoramic view will facilitate single-user-operability as, once fully acquired and spatially arranged, further frequent changes of views will no longer be required.

In an option, automatic presetting of the panoramic view is provided in order to reduce procedure time and increase its ease of use. Given the multiple degrees of freedom, the task of automatically achieving a desired panoramic view is non-trivial.

An important prerequisite for effectively utilizing the panoramic view is correctly identifying the relevant anatomical landmarks. Only then can the corresponding panoramic view be arranged smartly in a way that supports specific tasks. To minimize the inherent distortion encountered whenever a 3D sphere is being cut open and displayed as a flattened 3D image, the main region of interest may be located in the center of the panoramic view.

For example, in the Right Atrium (RA), in order to facilitate Coronary Sinus (CS) cannulation or Cavo-Tricuspid-Isthmus (CTI) line ablation, the CS orifice should be placed in the center. For transseptal procedure, the Fossa Ovalis (FO) should be centered whereas for Tricuspid Valve (TV) repair procedures, the valve should be centered. Similarly, to guide His Bundle Pacing, the Koch triangle should be fully exposed and the identified His site centered.

In the Left Atrium (LA), in order to facilitate Pulmonary Vein isolation (PVI), the two right septal and two left pulmonary veins should be arranged side-by-side when the ipsilateral veins are one on top of the other. This view will also expose the Left Atrial Appendage (LAA) ridge, which represents an Achilles hill of the procedure where gaps are often encountered. The LAA ridge can only be characterized by pre-acquired CT/MR and the system can image and display it in real-time. PV anatomical variations, like a common left PV, or three right PVs, will also be clearly identified without the need to perform angiography. If LAA occlusion is planned, then its orifice should be centered and if a Mitral Valve (MV) repair is intended, then the valve should be centered.

The algorithm takes into consideration the outlined constrains. In an example, based on artificial intelligence, the pre-specified anatomical landmarks are first identified based on geometrical data as well as electrophysiological information. Once identified, the algorithm finds the optimal combination of translation and rotation, as well as the location of the virtual camera, needed to generate the required panoramic view. The particular preset is thereafter displayed. Manual manipulation and further subtle changes are still permitted. In another example, the highlighted anatomical landmarks are also be fed back into the 3D reconstruction algorithm to improve the algorithm. For example, if the reconstruction algorithm merges two adjacent PVs or branches, the correct identification of these blood vessels by the new algorithm can overcome this and be used to better depict them as solitary vessels. This also facilitates improved depiction of PV ostia as well as the LAA ridge.

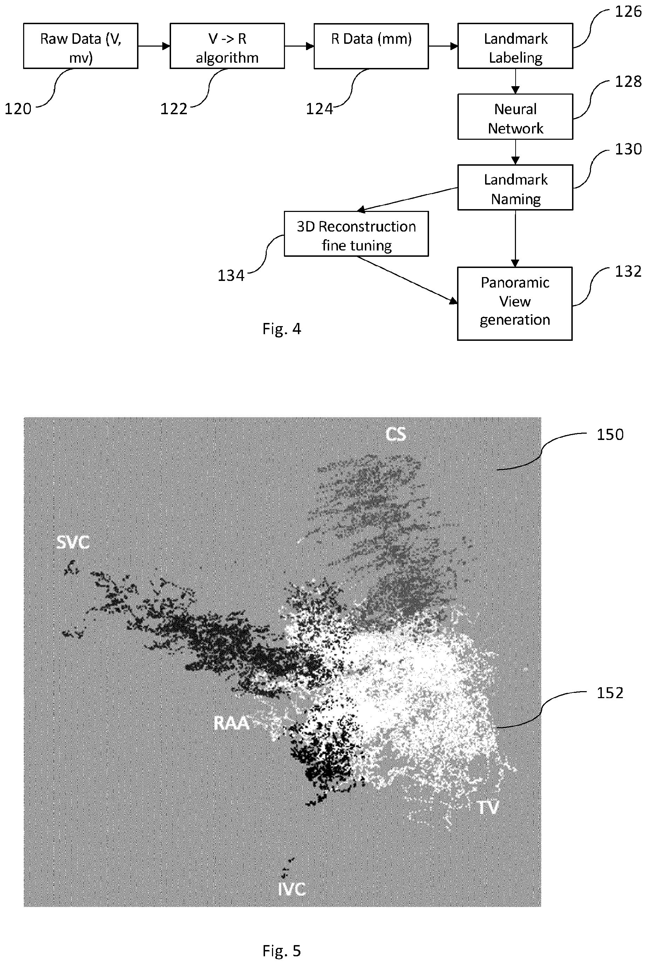

Fig. 4 shows another example of the method 100 for providing three-dimensional data of a hollow organ. In a first frame 120, raw data is provided. A second frame 122 indicated the volume to reality algorithm. A third frame 124 indicates a provision of raw data, such as providing millimeter values. A fourth frame 126 provides landmark labeling. The result is provided to a neural network 128. Further, a landmark naming 130 is provided. Next, a panoramic view generation 132 is provided. As an option, a 3D reconstruction fine tuning 134 is provided between the landmark naming 130 and the panoramic view generation 132.

For imaging a hollow organ, another example of an automatic panoramic view presetting algorithm is provided: Once a cardiac chamber is thoroughly imaged, its corresponding panoramic view facilitates self-explanatory spatial orientation, identification of all relevant anatomical landmarks (or anatomical variations) and execution of a specified planned procedure. The algorithm first identifies the characteristic anatomical landmarks of the particular chamber. Then it finds the optimal combination of translation and rotation, as well as the location of the virtual camera, needed to generate the required panoramic view. The particular preset is then displayed. Manual manipulation and further subtle changes are still permitted.

Fig. 5 shows an example of an interim state of a three-dimensional image data cloud 150 with measurements used for identifying anatomical landmarks. The three-dimensional image data cloud is indicated with a plurality of small spots 152 of measurement that were allocated to certain locations. The spots may be provided in different colors, for example relating to different segmented parts of the organ, to provide a better spatial understanding when regarding the data cloud.

In an example of an automatic anatomical landmark identification algorithm, each cardiac chamber is provided as a 3D ellipsoid using: (rx, ry, rz), (x0, y0, z0) and two rotation angles. In this example RA, landmark underwent labeling, identification and naming. This serves for both automatic presetting of the corresponding panoramic view as well as for sophisticated fine tuning of the 3D reconstruction.

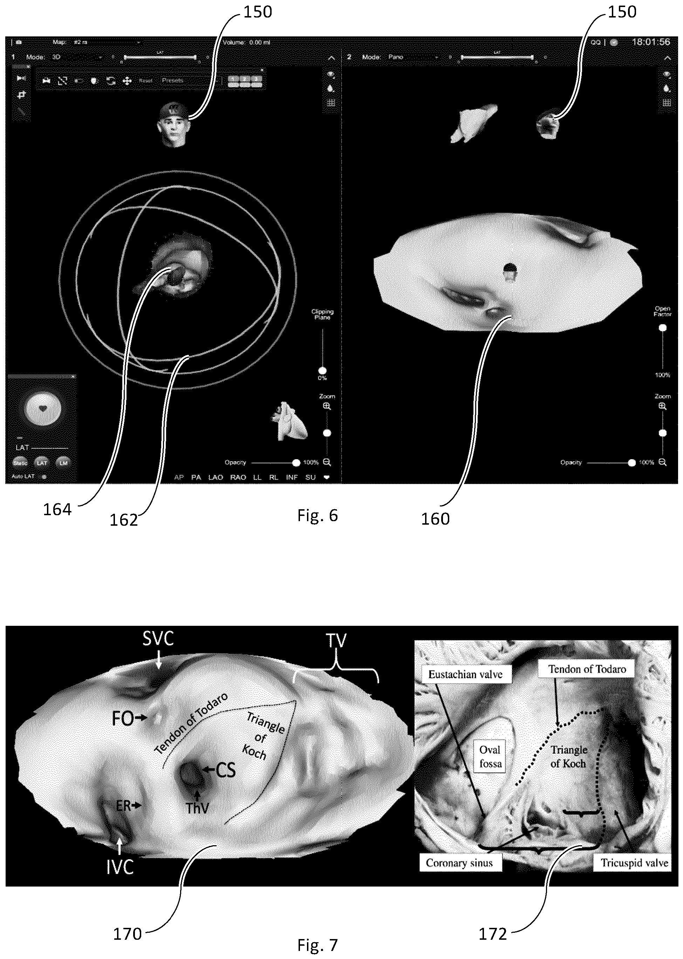

Fig. 6 shows an example of a setup for identifying a preferred viewing angle for projecting a flattened 3D view 160. Fig. 6 shows a panoramic view manual manipulation user interface. For example, reaching a desired arrangement of the anatomical landmarks of any imaged chamber is possible by manually controlling each degree of freedom at a time (by rotating colored orientation balls 162 or using the mouse directly on the panoramic view 160) as well as by amending the location of the virtual camera (small head icon 164). The left part indicates the degrees of freedom by circular lines in space around the result of the three-dimensional image data cloud.

Fig. 7 shows an example of a flattened panoramic view 170. Fig. 7 shows a right atrial (RA) panoramic view desired preset: Once the RA is thoroughly imaged, the panoramic view, which is a flattened 3D representation of the full 3D reconstruction shown in the left part of Fig. 7, facilitates self-explanatory spatial orientation, identification of all relevant anatomical landmarks (or anatomical variations) and execution of a planned procedure. This particular preset supports Atrial Flutter ablation and exposes the full Cavo-Tricuspid-Isthmus (CTI) line. It is also useful for fluoroless CS cannulation as the ThV is depicted. For physiological His Bundle Pacing (HBP), the Triangle of Koch must be fully exposed and the His be in the center where the inherent distortion is minimal in order to maintain maximal accuracy. Similarly, for transseptal procedure, the FO should be centered. Note the correlation between the RA panoramic view, shown on the left, and a surgical macroscopic view 172, shown on the right for explanation only. The following abbreviations are provided: SVC = Superior Vena Cava, IVC = Inferior Vena Cava, ER = Eustachian Ridge, CS = Coronary Sinus, ThV = Thebesian Valve, TV = Tricuspid Valve.

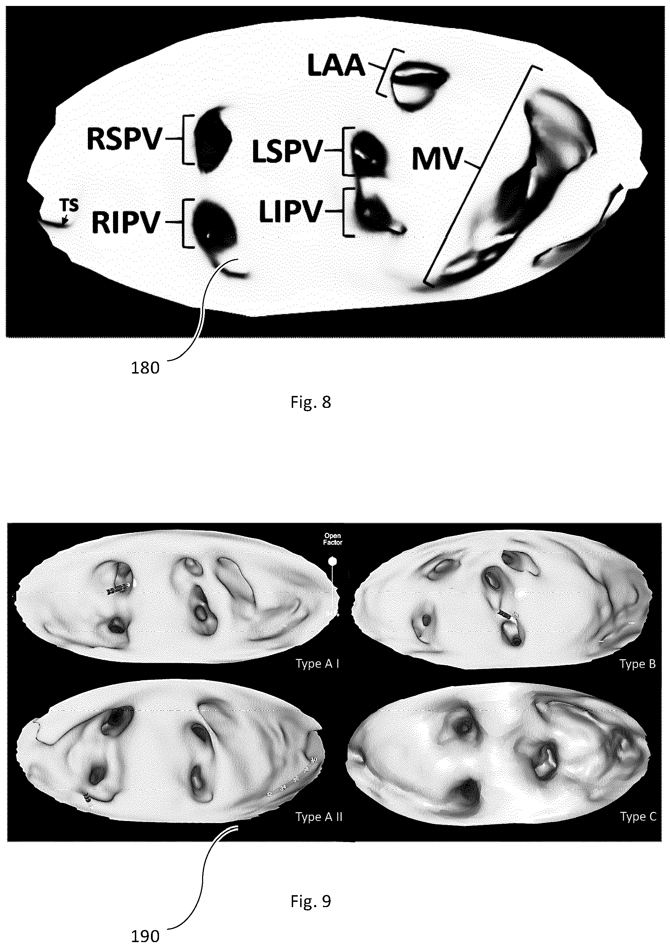

Fig. 8 shows another example of a flattened panoramic view 180. Fig. 8 shows a left atrial (LA) panoramic view desired preset: Once the LA is thoroughly imaged, the panoramic view facilitates self-explanatory spatial orientation, identification of all relevant anatomical landmarks (or anatomical variations) and execution of a specified planned procedure. This particular preset supports Atrial Fibrillation ablation - Pulmonary Vein Isolation (PVI). The LAA ridge, where gaps often occur, is depicted. Other presets are tailored for LAA occlusion procedures or Mitral Valve repair where the LAA orifice or the MV, respectively, should be placed in the center. The following abbreviations are provided: RSPV and LSPV = Right and Left Superior Pulmonary Veins, RIPV and LIPV = Right and Left Inferior Pulmonary Veins, LAA = Left Atrial Appendage, MV = Mitral Valve, TS = Transseptal Site.

Fig. 9 shows a further example of a flattened panoramic view 190. Fig. 9 shows left atrial appendage (LAA) ridge types: The previously described types, defined by virtual endoscopic view of the LAA ridge in cardiac CT, are depicted in LA panoramic view and it facilitates PVI ablation without gaps. In type A I (17.6%), the LAA ridge extends from the superior portion of the left superior PV ostium to the inferior portion of the left inferior PV ostium on the same plane with the inter-venous saddle between LSPV and LIPV. In type A II (69.9%), the LAA ridge is markedly prominent above the inter-venous saddle and extends from the superior portion of the LSPV to the inferior portion of the LIPV. In type B (5.9%), the ridge extends from the superior portion of the LSPV to the inter-venous saddle. Type C (6.6%): The LSPV and LIPV have a common trunk and the ridge runs from the upper edge of the trunk to the lower edge.

Examples for local electric field measurements inside a hollow organ can be found in WO 2018/130974 A1 and WO 2019/034944 A1.

In an example, the imaging of a hollow organ as described above is provided by, i.e. performed on an example of the KODEX system by Philips, for example on the Philips KODEX-EPD dielectric imaging and navigation system.

The term "subject" may also be referred to as individual. The "subject" may further also be referred to as patient, although it is noted that this term does not indicate whether any illness or disease is actually present with the subject.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for providing three-dimensional data of a hollow organ, comprising:
- a measurement input (12);
- a data processor (14); and
- an output interface (16);
wherein the measurement input is configured to receive a plurality of local electric field measurements (18) of at least one electrode on a catheter inserted in a lumen of a hollow organ of interest; and to receive geometrical data (20) representative of the location of the at least one electrode inside the lumen during the measurements;
wherein the data processor is configured to:
- receive pre-set electric field characteristics (22) associated with predetermined anatomical landmarks of the hollow organ expectable in the lumen in dependency of a type of the hollow organ;
- compare at least one of the plurality of local electric field measurements with the pre-set electric field characteristics to determine matching electric field measurements;
- based on the geometrical data, allocate local electric field measurements to matching electric field characteristics to identify anatomical landmarks of the hollow organ by identifying those local field measurements in the plurality of measurements that correspond to landmarks of the hollow organ;
- generate a three-dimensional image data cloud (24) by transforming the allocated electric field measurements into portions of the three-dimensional image data cloud based on the identified anatomical landmarks; and
wherein the output interface is configured to provide the three-dimensional image data cloud.

2. Device according to claim 1, wherein the data processor is configured to project a representation of an inner surface enclosing the lumen as a 3D view based on the three-dimensional image data cloud.

3. Device according to claim 1 or 2, wherein the data processor is configured to project a flattened 3D panoramic view of the inner surface enclosing the lumen based on the three-dimensional image data cloud, the flattened 3D panoramic view showing at least some surface parts of the inner surface; and
wherein the data processor is configured to provide a preset feature of interest in a center region of the flattened 3D panoramic view.

4. Device according claim 3, wherein the data processor is configured to generate the flattened 3D panoramic view based on user selected criteria;
wherein the selected criteria comprises a type of operation or treatment of the hollow organ; and
wherein for finding a projection according to the user selected criteria, the data processor is configured to arrange a plurality of different landmark related criteria in different weighting orders depending on the user selected criteria and to check if these criteria are fulfilled according to the respective weighting order for variations of projections.

5. Device according to one of the claim 2 to 4, wherein a display (26) is provided configured to display the 3D view and/or the flattened 3D panoramic view.

6. Device according to one of the preceding claims, wherein the pre-set electric field characteristics comprise at least one of the group of:
i) predefined gradients describing changes in the electric field; and
ii) predefined patterns describing attributes of the electric field; and
wherein, for the comparison, the data processor is configured to match the measured electric field values with the pre-set electric field characteristics to identify measurements relating to a location of a landmark.

7. A system (50) for measuring a hollow organ, the system comprising:
- a catheter (52) with at least one electrode;
- an electric field generator (54); and
- a device (10) for providing three-dimensional data of a hollow organ according to one of the preceding claims;
wherein the catheter is configured to be partly inserted into the lumen and moved inside the lumen; and to conduct a plurality of local electric field measurements inside the lumen with the at least one electrode; and to provide the local electric field measurements data to the measurement input of the device for providing three-dimensional data of a hollow organ; and
wherein the electric field generator is configured to generate at least one electric field inside a lumen of a hollow organ, and to provide the geometrical data representative of the location of the at least one electrode inside the lumen during the measurements to the measurement input of the device for providing three-dimensional data of a hollow organ.

8. System according to claim 7, wherein it is further provided at least one interventional device for insertion into a hollow organ;
wherein the interventional device comprises at least one tool for treatment inside the hollow organ;
wherein the interventional device comprises the catheter having at least one electrode; and
wherein the catheter is provided for guiding the at least one tool inside the hollow organ.

9. System according to claim 7 or 8, wherein the geometrical data comprises spatial tracking of the at least one electrode inside the organ during the measurements by the electric field generator; and
wherein a tracker is provided configured to provide spatial information of a starting point for the moving of the catheter inside the hollow organ; and wherein the spatial tracking is based on the starting point.

10. System according to one of the claims 7 to 9, wherein the catheter is configured to be inserted into:
- a heart comprising several chambers as the hollow organ, from which heart at least one chamber is to be imaged; and/or
- at least one of the group of bladder, uterus, stomach and colon.

11. A method (100) for providing three-dimensional data of a hollow organ, the method comprising the following steps:
- receiving (102) a plurality of local electric field measurements of at least one electrode on a catheter inserted in a lumen of a hollow organ of interest;
- providing (104) geometrical data representative of the location of the at least one electrode inside the lumen during the measurements;
- providing (106) pre-set electric field characteristics associated with predetermined anatomical landmarks of the hollow organ expectable in the lumen in dependency of a type of the hollow organ;
- comparing (108) at least one of the plurality of local electric field measurements with the pre-set electric field characteristics to determine matching electric field measurements;
- allocating (110) local electric field measurements to matching electric field characteristics based on the geometrical data to identify anatomical landmarks of the hollow organ by identifying those local field measurements in the plurality of measurements that correspond to landmarks of the hollow organ;
- generating (112) a three-dimensional image data cloud by transforming the allocated electric field measurements into portions of the three-dimensional image data cloud based on the identified anatomical landmarks; and
- outputting (114) the three-dimensional image data cloud.

12. Method according to claim 11, further comprising:
- inserting a catheter with at least one electrode in a lumen of a hollow organ and moving the at least one electrode inside the lumen; and
- conducting a plurality of local electric field measurements inside the lumen with the at least one electrode for providing the local electric field measurements.

13. Method according to claim 11 or 12, wherein it is further provided:
- projecting (116) a representation of an inner surface enclosing the lumen as a 3D view based on the three-dimensional image data cloud; and/or
- projecting (118) a flattened 3D panoramic view of the inner surface enclosing the lumen based on the three-dimensional image data cloud, the flattened 3D panoramic view showing at least some surface parts of the inner surface; wherein a preset feature of interest is provided in a center region of the flattened 3D panoramic view.

14. A computer program enabling a processor to carry out the method of one of the claims 11 to 13.

15. A computer readable medium having stored the program element of claim 14.
